(19) Europäisches Patentamt
European Patent Office
Office européen des brevets

(11) EP 2 540 278 A1

(12) EUROPEAN PATENT APPLICATION
published in accordance with Art. 153(4) EPC

(43) Date of publication:
**02.01.2013 Bulletin 2013/01**

(21) Application number: **11747370.2**

(22) Date of filing: **23.02.2011**

(51) Int Cl.:
***A61K 8/41*** *(2006.01)* ***A61K 8/34*** *(2006.01)*
***A61K 8/42*** *(2006.01)* ***A61K 8/46*** *(2006.01)*
***A61K 8/73*** *(2006.01)* ***A61Q 5/04*** *(2006.01)*

(86) International application number:
**PCT/JP2011/053937**

(87) International publication number:
**WO 2011/105410 (01.09.2011 Gazette 2011/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.02.2010 JP 2010043094**

(71) Applicant: **SHISEIDO COMPANY, LTD.**
**Chuo-ku,**
**Tokyo 104-8010 (JP)**

(72) Inventors:
- **YAMAKI Satoshi**
  **Yokohama-shi**
  **Kanagawa 224-8558 (JP)**
- **KAWADA Emiko**
  **Yokohama-shi**
  **Kanagawa 224-8558 (JP)**

(74) Representative: **Schön, Christoph**
**Dr. Schön & Partner**
**Bavariaring 26**
**80336 München (DE)**

# (54) HAIR STYLING COMPOSITION

(57) Provided is a hair shape-controlling composition containing a reducing agent and an alkaline agent, which composition contains (1) a hydroxy-ether-amine compound (I) (wherein $R^1$ represents a linear or branched $C_{6-24}$ alkyl or alkenyl group; $R^2$ represents a linear or branched $C_{1-6}$ hydroxyalkylene or hydroxyalkylenyl group; and each of $R^3$ and $R^4$, which may be identical to or different from each other, represents a hydrogen atom or a linear $C_{1-6}$ alkyl group); (2) an amidoamine compound (II) (wherein $R^5$ represents a linear or branched $C_{6-24}$ alkyl or alkenyl group; A represents -CONH-; $R^6$ represents a linear or branched $C_{1-6}$ alkylene or alkylenyl group; and each of $R^7$ and $R^8$, which may be identical to or different from each other, represents a hydrogen atom or a linear or branched $C_{1-6}$ alkyl group) and/or O-[2-hydroxy-3-(trimethylammonio)propyl]hydroxyethylcellulose chloride; and (3) a higher alcohol. The hair shape-controlling composition exhibits greatly improved hair shape-controlling performance.

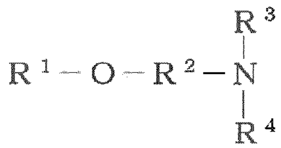
(I)

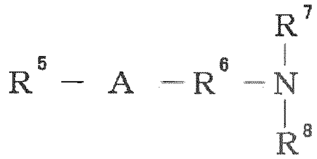
(II)

EP 2 540 278 A1

**Description**

Technical Field

The present invention relates to a composition for controlling the shape of hair (hereinafter may be referred to as a "hair shape-controlling composition"). More particularly, the present invention relates to a composition adopted in hair treatment for the purpose of controlling the shape of hair, such as permanent wave treatment or hair straightening treatment.

Background Art

Permanent wave treatment or hair straightening treatment has been performed for intentionally controlling the shape of hair for the sake of fashion or appearance.

In many cases, permanent wave treatment or hair straightening treatment is carried out by use of a "two-component hair shape-controlling composition"; specifically, such a hair treatment is performed by applying, to hair, a first component for permanent waving or hair straightening containing a reducing agent (e.g., thioglycolic acid or a salt thereof, thiolactic acid or a salt thereof, or cysteine or a salt thereof) and an alkaline agent (e.g., aqueous ammonia, monoethanolamine, or ammonium hydrogencarbonate), thereby reduction-cleaving disulfide bonds present in proteins forming the hair and attaining a desired hair shape, and then treating the hair with a second component containing, as a main component, an oxidizing agent (e.g., sodium bromate or hydrogen peroxide), thereby re-forming disulfide bonds at positions different from the original positions.

In recent years, such a hair treatment has also been carried out by use of a "one-component hair shape-controlling composition," in which natural (air) oxidation is adopted in place of oxidation with a second component.

Generally, when hair undergoes permanent wave treatment or hair straightening treatment, the thus-treated hair tends to be damaged (i.e., the hair loses smoothness, fingers do not smoothly run through the hair, the hair becomes rough, or the hair loses moisture), and the hair shows a poor texture. In addition, since the surface of the treated hair is roughened, the hair loses a glossy appearance; i.e., the hair has an appearance markedly different from that of healthy hair, and thus the treated hair tends to make a strong impression of being apparently damaged.

In order to solve such a problem, the present applicant previously conducted technical studies, and provided a hair shape-controlling composition which has excellent applicability to hair, which causes less damage to permanent-waved hair or straightened hair, which can provide treated hair with an excellent texture, and which does not impede a glossy appearance of treated hair, the composition being produced by incorporating a specific surfactant into a hair shape-controlling composition containing a reducing agent and an alkaline agent (i.e., a composition for permanent wave treatment or hair straightening treatment) (Patent Document 1).

Prior Art Document

Patent Document

Patent Document 1: Japanese Patent Application Laid-Open (*kokai*) No. 2008-231098
Patent Document 2: Japanese Patent Application Laid-Open (kokai) No. 2004-323495
Patent Document 3: Japanese Patent Application Laid-Open (kokai) No. 2004-323496

Summary of the Invention

Problems to be Solved by the Invention

An object of the present invention is to technically develop the hair shape-controlling composition disclosed in Patent Document 1, to thereby provide means for further improving hair shape-controlling performance.

Means for Solving the Problems

The present inventors have found that when a specific hydroxy-ether-amine compound, a specific amidoamine compound and/or cationized cellulose, and a higher alcohol are incorporated into a hair shape-controlling composition, the resultant hair shape-controlling composition causes much less damage to hair during use thereof, provides hair with a favorable texture, and realizes further improved hair handling performance and hair straightening performance. The

present invention has been accomplished on the basis of this finding.

Accordingly, the present invention provides a hair shape-controlling composition comprising a reducing agent and an alkaline agent, which composition is characterized by comprising the following components (1) to (3):

(1) a hydroxy-ether-amine compound represented by the following formula (I):

[F1]

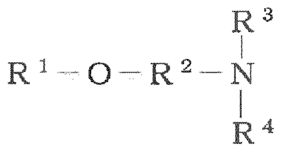

(I) ,

wherein $R^1$ represents a linear or branched C6 to C24 alkyl or alkenyl group; $R^2$ represents a linear or branched C1 to C6 hydroxyalkylene or hydroxyalkylenyl group; and each of $R^3$ and $R^4$, which may be identical to or different from each other, represents a hydrogen atom or a linear C1 to C6 alkyl group;

(2) an amidoamine compound represented by the following formula (II) and/or O-[2-hydroxy-3-(trimethylammonio) propyl]hydroxyethylcellulose chloride;

[F2]

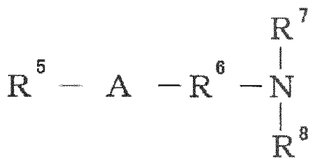

(II) ,

wherein $R^5$ represents a linear or branched C6 to C24 alkyl or alkenyl group; A represents an amide bond (-CONH-); $R^6$ represents a linear or branched C1 to C6 alkylene or alkylenyl group; and each of $R^7$ and $R^8$, which may be identical to or different from each other, represents a hydrogen atom or a linear or branched C1 to C6 alkyl group; and

(3) a higher alcohol (hereinafter the hair shape-controlling composition may be referred to as "the present composition").

The effects of the present composition can be further improved by incorporating a sugar alcohol and/or monoethanolamine. In one referred mode, the present composition comprises a quaternary ammonium salt. In another preferred mode, the present composition is adopted as a first component of a two-component hair shape-controlling composition which comprises the first component containing a reducing agent and an alkaline agent, and a second component containing an oxidizing agent.

When the present composition is a first component of a two-component hair shape-controlling composition, the amount of each component of the present composition represents that on the basis of the first component of the two-component hair shape-controlling composition, whereas the present composition is a one-component hair shape-controlling composition, the amount of each component of the present composition represents that on the basis of the entire one-component hair shape-controlling composition.

Effects of the Invention

According to the present invention, there is provided a hair shape-controlling composition which has excellent applicability to hair during use thereof for controlling the shape of the hair, which causes much less damage to permanent-waved hair or straightened hair, which can provide treated hair with a further excellent texture, which does not impede a glossy appearance of treated hair, and which realizes further improved hair handling performance and hair straightening performance.

Brief Description of the Drawings

[Fig. 1]
Fig. 1 shows the results of a combing test performed on the invention product.
[Fig. 2]
Fig. 2 shows the results of a hair tensile test performed on the invention product.

Best Modes for Carrying Out the Invention

[Essential component of the present composition]

(1) Reducing agent and alkaline agent

The reducing agent and alkaline agent contained in the present composition are components for cleaving disulfide bonds in hair.
Examples of the reducing agent include thiolactic acid or a salt thereof, thioglycolic acid or a salt thereof, cysteine or a salt thereof, acetylcysteine or a salt thereof, cysteamine or a salt thereof, and cyclic mercapto compounds.
The present composition may contain, as a preferred reducing agent, thiolactic acid or a salt thereof, which exhibits an excellent effect of straightening curly hair during hair straightening. Examples of the thiolactic acid salt include an ammonium salt and a monoethanolamine salt of thiolactic acid. The amount of thiolactic acid or a salt thereof contained in the present composition is preferably 1 to 12 mass%, particularly preferably 2 to 11 mass%.
Examples of the alkaline agent include monoethanolamine, ammonia, inorganic ammonium salts (e.g., ammonium hydrogencarbonate), organic ammonium salts, organic amines, inorganic alkaline agents, and basic amino acids.
Of these, monoethanolamine is most preferably incorporated in the present composition. In such a case, when the composition is applied to hair, the hair swells, and the reducing agent can readily permeate the swollen hair.
When monoethanolamine is incorporated in the present composition, the amount of monoethanolamine is preferably 0.01 to 8 mass%, particularly preferably 0.1 to 4 mass%, on the basis of the entirety of the composition. When the amount is less than 0.01 mass% on the basis of the entirety of the composition, the effect of incorporation of monoethanolamine is less likely to be obtained, whereas when the amount exceeds 8 mass% on the basis of the entirety of the composition, the resultant composition tends to cause more damage to hair.
The reducing agent and the alkaline agent may be appropriately chosen in consideration of, for example, the specific intended use (for controlling the shape of hair) of the present composition, or the types of the coexistent reducing agent and alkaline agent.

(2) Surfactant of formula (I)

The present composition contains a hydroxy-ether-amine compound represented by formula (I) (hereinafter may be referred to as "hydroxy-ether-amine compound (I)").
The hydroxy-ether-amine compound (I) may be produced through a known production method (see, for example, Patent Document 2 or 3), or may be a commercially available product (e.g., Catinal SHPA-80: N-(2-hydroxy-3-stearoxypropyl)-N,N-dimethylamine, product of Toho Chemical Industry Co., Ltd.).
In the formula representing the hydroxy-ether-amine compound (I), $R^1$ represents a linear or branched C6 to C24 alkyl or alkenyl group. Examples of the linear or branched C6 to C24 alkyl or alkenyl group include hexyl, heptyl, octyl, nonyl, decyl, undecyl, dodecyl, tridecyl, tetradecyl, pentadecyl, hexadecyl, heptadecyl, stearyl (octadecyl), nonadecyl, icosyl, henicosyl, docosyl, tricosyl, tetracosyl, myristoleyl, palmitoleyl, oleyl, linoyl, linoleyl, ricinoleyl, and isostearyl. Examples of the linear or branched C1 to C6 hydroxyalkylene or hydroxyalkylenyl group (divalent group) represented by $R^2$ include hydroxyethylene, hydroxytrimethylene, hydroxytetramethylene, hydroxypentamethylene, and hydroxyhexamethylene. As described above, each of $R^3$ and $R^4$, which may be identical to or different from each other, represents a hydrogen atom or a linear C1 to C6 alkyl group.
In the present invention, the aforementioned N-(2-hydroxy-3-stearoxypropyl)-N,N-dimethylamine is a particularly preferred hydroxy-ether-amine compound (I), in which $R^1$ is a stearyl group (octadecyl group), $R^2$ is a 2-hydroxypropylene group (divalent group), and each of $R^3$ and $R^4$ is a methyl group.
The amount of the hydroxy-ether-amine compound (I) contained in the present composition is preferably about 0.01 to about 8 mass%, particularly preferably 0.1 to 4 mass%, on the basis of the entirety of the composition.
When the amount of the hydroxy-ether-amine compound (I) is less than 0.01 mass% on the basis of the entirety of the present composition, hair treated with the composition tends to lack smooth texture during rinsing or after drying and to exhibit poor gloss, whereas when the amount of the hydroxy-ether-amine compound (I) exceeds 8 mass% on the basis of the entirety of the composition, the composition itself poses a problem in terms of stability, and tends to lack spreadability during application thereof to hair.

(3) Amidoamine compound and O-[2-hydroxy-3-(trimethylammonio)propyl]hydroxyethylcellulose chloride

(a) Amidoamine compound represented by formula (II) (hereinafter may be referred to as "amidoamine compound (II)")

In the formula representing the amidoamine compound (II), $R^5$ represents a linear or branched C6 to C24 alkyl or alkenyl group. Examples of the linear or branched C6 to C24 alkyl or alkenyl group include hexyl, heptyl, octyl, nonyl, decyl, undecyl, dodecyl, tridecyl, tetradecyl, pentadecyl, hexadecyl, heptadecyl, stearyl (octadecyl), nonadecyl, icosyl, henicosyl, docosyl, tricosyl, tetracosyl, myristoleyl, palmitoleyl, oleyl, linoyl, linoleyl, ricinoleyl, and isostearyl. Examples of the linear or branched C1 to C6 alkylene or alkylenyl group represented by $R^6$ include methylene, ethylene, trimethylene, tetramethylene, pentamethylene, and hexamethylene. As described above, each of $R^7$ and $R^8$, which may be identical to or different from each other, represents a hydrogen atom or a linear or branched C1 to C6 alkyl group (typically a methyl group).

Among the above-exemplified amidoamine compounds (II), the particularly preferred amidoamine compounds (II) are stearic acid dimethylaminopropylamide and stearic acid diethylaminoethylamide.

Amidoamine compounds (II) may be used singly or in combination of two or more species. The amount of the amidoamine compound(s) (II) is preferably about 0.01 to about 8 mass%, particularly preferably 0.1 to 4 mass%, on the basis of the entirety of the composition. When the amount of the amidoamine compound(s) (II) is less than 0.01 mass% on the basis of the entirety of the composition, the present composition may fail to sufficiently exhibit the effect of suppressing damage to hair, or may fail to realize sufficiently easy hair handling performance. In contrast, even when the amount of the amidoamine compound(s) (II) exceeds 8 mass% on the basis of the entirety of the composition, an effect commensurate with an increase in amount is less likely to be attained.

(b) O-[2-hydroxy-3-(trimethylammonio)propyl]hydroxyethylcellulose chloride (hereinafter may be referred to as "cationized cellulose")

O- 2-hydroxy-3-(trimethylammonio)propyl]hydroxyethylcellulose chloride (cosmetic ingredient name: Polyquaternium-10) is currently adopted as a hair conditioning component.

The amount of O-[2-hydroxy-3-(trimethylammonio)propyl]hydroxyethylcellulose chloride is preferably 0.01 to 4 mass%, particularly preferably 0.1 to 2 mass%, on the basis of the entirety of the composition. When the amount of the compound is less than 0.01 mass% on the basis of the entirety of the composition, the present composition may fail to sufficiently exhibit the effect of suppressing damage to hair, or may fail to realize sufficiently easy hair handling performance. In contrast, even when the amount of the compound exceeds 2 mass% on the basis of the entirety of the composition, an effect commensurate with an increase in amount is less likely to be attained.

(c) The present composition may contain either or both of the aforementioned amidoamine compound (II) and cationized cellulose.

(4) Higher alcohol

No particular limitation is imposed on the higher alcohol which may be incorporated into the present composition. Examples of the higher alcohol include lauryl alcohol, myristyl alcohol, cetyl alcohol, cetostearyl alcohol, stearyl alcohol, aralkyl alcohol, behenyl alcohol, and jojoba alcohol. Of these, preferred higher alcohols are cetyl alcohol and stearyl alcohol.

The amount of the higher alcohol contained in the present composition is preferably 0.1 to 20 mass%, particularly preferably 1 to 10 mass%, on the basis of the entirety of the composition. When the amount of the higher alcohol is less than 0.1 mass% on the basis of the entirety of the composition, hair treated with the composition tends to lack smooth texture, whereas when the amount of the higher alcohol exceeds 20 mass%, the base becomes excessively hard, and many manufacturing problems tend to arise.

[Optional component of the present composition]

(5) Sugar alcohol

When a sugar alcohol is incorporated into the present composition, the composition further exhibits the effect of suppressing damage to hair, and exhibits further improved hair handling performance.

No particular limitation is imposed on the sugar alcohol which may be incorporated into the present composition. Examples of the sugar alcohol include maltitol, mannitol, sorbitol, and xylitol. Of these, sorbitol is particularly preferred.

When a sugar alcohol is incorporated into the present composition, the amount of the sugar alcohol is preferably

1 to 40 mass%, particularly preferably 5 to 20 mass%, on the basis of the entirety of the composition. When the amount of the sugar alcohol is less than 1 mass% on the basis of the entirety of the composition, the effect of incorporation of the sugar alcohol is less likely to be obtained, whereas when the amount of the sugar alcohol exceeds 40 mass% on the basis of the entirety of the composition, the composition greatly tends to become sticky.

(6) Quaternary ammonium salt

When a quaternary ammonium salt is incorporated into the present composition, the composition exhibits reduced skin irritation and improved washing performance and foaming performance. Examples of the quaternary ammonium salt which may be incorporated include alkyltrimethylammonium salts and alkyldimethylaminoacetic acid betaines. The alkyl group of such a quaternary ammonium salt may be selected from among alkyl groups of quaternary ammonium salts which are generally incorporated into hair cosmetic compositions. Specific examples of the alkyl group include lauryl, myristyl, palmityl, stearyl, and behenyl. The present composition can preferably contain behenyltrimethylammonium chloride or stearyltrimethylammonium chloride.

When a quaternary ammonium salt is incorporated into the present composition, no particular limitation is imposed on the amount of the quaternary ammonium salt. However, generally, the amount of the quaternary ammonium salt is preferably 0.01 to 5 mass% on the basis of the entirety of the composition.

The present composition may optionally contain, in addition to the aforementioned components, an appropriate amount of a component which may be generally adopted in a permanent waving agent composition or a hair straightening agent composition.

Examples of the optionally incorporated component include water; humectants (e.g., propylene glycol, dipropylene glycol, and glycerin); water-soluble polymer compounds (e.g., methylcellulose and hydroxyethylcellulose); cationic polymer compounds (e.g., a copolymer of a methacryloxyethyltrimethylammonium salt and polyvinyl pyrrolidone); water-soluble silicone; nonionic surfactants (e.g., polyoxyethylene hydrogenated castor oil and polyoxyethylene alkyl ether); amphoteric surfactants (e.g., 2-alkyl-N-carboxymethyl-N-hydroxyethylimidazolinium betaine and trialkylaminoacetic acid betaine); penetrants (e.g., urea, alkyl urea, benzyl alcohol, and monoethylene oxide benzyl ether); natural animal (or plant) extracts; amino acids; organic acids (e.g., citric acid and lactic acid); inorganic salts (e.g., sodium chloride and potassium chloride); perfumes; preservatives (e.g., paraben); sequestering agents (e.g., EDTA-3Na); UV absorbents (e.g., oxybenzone); and colorants.

No particular limitation is imposed on the product form of the present composition, and the composition may be provided in the form of non-emulsified product or emulsified product (oil-in-water type or water-in-oil type). The present composition in the form of, for example, liquid, emulsion, or cream may be produced through a customary method.

As described above, when the present composition is used for attaining a desired hair shape, generally, a process corresponding to a two-component composition is carried out. Firstly, the first component of the hair shape-controlling composition containing a reducing agent and an alkaline agent may be brought into contact with hair, and subsequently the second component containing an oxidizing agent may be brought into contact with the hair, to thereby control the shape of the hair. Alternatively, there may be carried out a process corresponding to a one-component composition, in which natural (air) oxidation is adopted, and use of the second component containing an oxidization agent is omitted.

As described above, the expression "hair shape control" encompasses permanent wave treatment and hair straightening treatment.

The second component of a two-component composition generally contains an oxidizing agent. Examples of the oxidizing agent include compounds containing a peroxide such as hydrogen peroxide, sodium peroxocarbonate, or urea peroxide; alkali metal peracid salts such as sodium perborate, sodium persulfate, and potassium monopersulfate; and products containing an oxidizing agent such as an alkali metal bromate (e.g., sodium bromate or potassium bromate). No particular limitation is imposed on the amount of the oxidizing agent contained in the second component, and the amount may be appropriately determined in consideration of, for example, the specific intended use (for controlling the shape of hair) of the present composition, or the type of the oxidizing agent adopted.

In any of the aforementioned hair shape-controlling processes, a step of physically fixing hair (typically, a step of winding hair around a curler for permanent waving, or a step of fixing hair by means of a hair straightening instrument for attaining a desired hair shape), a washing step, a heating step by means of a hair dryer, and so on, may be appropriately carried out according to need.

The permanent waving process may be carried out through, for example, the following steps (1) to (4):

(1) hair fibers are fixed by winding them around a curler;
(2) a first component containing a reducing agent is applied to the hair fibers, and the hair fibers are allowed to stand for 15 to 30 minutes;
(3) the first component is rinsed off with water while the hair fibers are fixed, and a second component containing

an oxidizing agent is applied to the hair fibers, followed by allowing them to stand for 5 to 20 minutes; and
(4) the fixing instrument is removed, and the second component is rinsed off with running water, followed by drying of the hair fibers with a dryer for finishing.

In this process, step (3) (i.e., application of the second component containing an oxidizing agent) may be omitted.
The hair straightening process may be carried out through, for example, the following steps (1) to (5):

(1) a first component containing a reducing agent is applied to hair fibers, and then the hair fibers are allowed to stand for 15 to 30 minutes;
(2) the first component is rinsed off with running water, and the hair fibers are dried with a dryer;
(3) the hair fibers are straightened with a hair iron (in this case, preferably, bath treatment is carried out twice on a handful of hair at 140 to 180°C);
(4) a second component containing an oxidizing agent is applied to the hair fibers, and the hair fibers are allowed to stand for 5 to 20 minutes; and
(5) the second component is rinsed off with running water, and the hair fibers are dried with a dryer for finishing.

Similar to the case of the permanent waving process, step (4) (i.e., application of the second component containing an oxidizing agent) may be omitted.

Examples

he present invention will next be described in more detail by way of examples, which should not be construed as limiting the invention thereto. Unless otherwise specified, the amount of a component incorporated is represented by "mass%." The amounts of components in a first component and a second component are described on the basis of the first component and the second component, respectively.

[Formulation of second component]

The second component of a hair shape-controlling composition adopted in the below-described Test Examples 1 to 3 was prepared by mixing the components shown in Table 1.
[Table 1]

| Component | Amount (mass%) |
|---|---|
| Aqueous hydrogen peroxide | 1.5 |
| Mineral oil | 0.3 |
| Cetyl alcohol | 2.0 |
| Stearyltrimonium chloride | 1.0 |
| Pentasodium diethylenetriaminepentaacetate | Appropriate amount |
| Phosphoric acid | Appropriate amount |
| Disodium hydrogenphosphate dodecahydrate | Appropriate amount |
| Purified water | Balance |
| Total | 100 |

[Test Example 1] Actual use test

<Test method>

For evaluation of the effects of the present invention, in the present Example, hair samples were subjected to a test employing permanent wave treatment or hair straightening treatment.

(1) Permanent wave treatment

A plurality of hair bundles (each having a weight of 0.7 g and a length of 20 cm) (hereinafter may be referred to as hair samples) were prepared from non-chemically damaged hair fibers collected from a single person. The thus-prepared hair samples were immersed in a commercially available breaching agent (ammonia: 0.8%, hydrogen peroxide: 3.0%, potassium persulfate: 0.1%) for 20 minutes so that the hair was damaged to the same extent. Each of the thus-bleached hair samples was wound around a plastic rod having a diameter of 1.5 cm, and a first component (2.0 mL) was applied to the hair sample. The hair sample was allowed to stand at 25°C for 15 minutes and then washed with water. Subsequently, a second component (2.0 mL) was applied to the hair sample, and the hair sample was allowed to stand at 25°C for 15 minutes and then washed with water, followed by natural drying.

(2) Hair straightening treatment

In a manner similar to the case of the permanent wave treatment described above in (1), bleached hair samples were prepared. The first component (2.0 g) was applied to each of the bleached hair samples, and the hair sample was allowed to stand at 25°C for 15 minutes and then washed with water, followed by drying with a towel. Thereafter, the thus-dried hair sample was treated with a high-temperature styling iron set at 170°C for three seconds. Subsequently, the second component (2.0 g) was applied to the hair sample, and the hair sample was allowed to stand at 25°C for five minutes and then washed with water, followed by natural drying.

(3) Evaluation

(a) Damage of hair

The degree of damage of hair after the final natural drying in the aforementioned treatment (1) or (2) was organoleptically evaluated by 12 expert panelists. Specifically, when six of the 12 expert panelists recognized a significant difference, rating "±1" was assigned, whereas when nine of the 12 expert panelists recognized a significant difference, rating "±2" was assigned. A positive number (+) represents a tendency toward improvement, and a negative number (-) represents a tendency toward deterioration.

(b) Damage sensation by hair texture

A hair sample which had undergone the aforementioned treatment (1) or (2) was subjected to an actual use test (in terms of damage sensation by hair texture) by the 12 expert panelists. When six of the 12 expert panelists recognized a significant difference, rating "±1" was assigned, whereas when nine of the 12 expert panelists recognized a significant difference, rating "±2" was assigned. A positive number (+) represents a tendency toward improvement, and a negative number(-) represents a tendency toward deterioration.

(c) Hair straightening effect

A hair sample after the final natural drying in the aforementioned treatment (2) was subjected to an actual use test by the 12 expert panelists for evaluation of a hair straightening effect (generally in terms of easy hair handling performance). When six of the 12 expert panelists recognized a significant difference, rating "±1" was assigned, whereas when nine of the 12 expert panelists recognized a significant difference, rating "±2" was assigned. A positive number (+) represents a tendency toward improvement, and a negative number(-) represents a tendency toward deterioration.

(d) Panelist acceptability

The acceptability of a hair sample which had undergone the aforementioned treatment (1) or (2) was evaluated by the 12 expert panelists. When six of the 12 expert panelists recognized a significant difference, rating "±1" was assigned, whereas when nine of the 12 expert panelists recognized a significant difference, rating "±2" was assigned. A positive number (+) represents a tendency toward improvement of acceptability, and a negative number(-) represents a tendency toward deterioration of acceptability.

<Results of Test Example 1>

Test samples employed in Test Example 1 (Examples 1 to 4 and Comparative Examples 1 and 2) were prepared according to formulations shown in Table 2. Specifically, each test sample was prepared by mixing surfactants and oily

components with ion-exchanged water heated to 75°C, cooling the resultant mixture to 40°C or lower, and adding a reducing agent, a stabilizer, and a perfume to the mixture.

For evaluation of the thus-prepared test samples, they were subjected to the aforementioned evaluation tests (a) to (d). The below-described "stearoxyhydroxypropylamine" is an abbreviation of N-(2-hydroxy-3-stearoxypropyl)-N, N-dimethylamine, which is a hydroxy-ether-amine compound represented by formula (I) (hydroxy-ether-amine compound (I)) wherein $R^1$ is a stearyl group (octadecyl group), $R^2$ is a 2-hydroxypropylene group (divalent group), and each of $R^3$ and $R^4$ is a methyl group.

[Table 2]

| | Comp. Ex. 1 | Comp. Ex. 2 | Ex. 1 | Ex. 2 | Ex. 3 | Ex. 4 |
|---|---|---|---|---|---|---|
| Component | Amount (mass%) | | | | | |
| Purified water | Balance | Balance | Balance | Balance | Balance | Balance |
| Aminopropyl dimethvcone | 1.0 | 1.0 | 1.0 | 1.0 | 1.0 | 1.0 |
| Mineral oil | 1.5 | 1.5 | 1.5 | 1.5 | 1.5 | 1.5 |
| Cetyl alcohol | 5.0 | 5.0 | 5.0 | 5.0 | 5.0 | 5.0 |
| Glycerin | 10.0 | 10.0 | 10.0 | - | - | - |
| Sorbitol | - | - | - | 10.0 | 10.0 | 10.0 |
| Propylene glycol | 3.0 | 3.0 | 3.0 | 3.0 | 3.0 | 3.0 |
| Polyoxyethylene oleyl ether | 0.5 | 0.5 | 0.5 | 0.5 | 0.5 | 0.5 |
| Behentrimonium chloride | 2.0 | 1.2 | 1.0 | 1.0 | 1.0 | 1.0 |
| Stearoxyhydroxyoropylamine | - | 0.5 | 0.5 | 0.5 | 0.5 | 0.5 |
| Stearic acid dimethylaminopropylamide | - | - | 0.3 | 0.3 | - | - |
| Stearic acid diethylaminoethylamide | - | - | - | - | 0.3 | - |
| Monoethanolamine | 1.0 | 1.0 | 1.0 | 1.0 | 1.0 | 1.0 |
| Ammonium thioglycolate | 3.5 | - | - | - | - | - |
| Ammonium thiolactate | - | 3.5 | 3.5 | 3.5 | 3.5 | 3.5 |
| Diammonium dithiodiglycolate | 3.5 | 2.5 | 2.5 | 2.5 | 2.5 | 2.5 |
| O-[2-hydroxy-3-(trimethylammonio) propyl]-hydroxyethylcellulose chloride | - | - | - | - | 0.5 | 0.5 |
| Pentasodium diethylenetriaminepentaacetate | Appropriate amount | Appropriate amount | Appropriate amount | Appropriate amount | Appropriate amount | Appropriate amount |
| Perfume | Appropriate amount | Appropriate amount | Appropriate amount | Appropriate amount | Appropriate amount | Appropriate amount |

(continued)

| | Comp. Ex. 1 | Comp. Ex. 2 | Ex. 1 | Ex. 2 | Ex. 3 | Ex. 4 |
|---|---|---|---|---|---|---|
| Component | Amount (mass%) | | | | | |
| Total | 100 | 100 | 100 | 100 | 100 | 100 |
| Test Results | | | | | | |
| Damage of hair | 0 | 1 | 1 | 1 | 1 | 1 |
| Damage sensation by hair texture | 0 | 0 | 1 | 2 | 1 | 1 |
| Hair straightening effect (easy hair handling performance) | 0 | -1 | 1 | 2 | 2 | 2 |
| Panelist acceptability | 0 | 0 | 1 | 1 | 1 | 1 |

As shown in the data of Table 2, the present compositions (Examples 1 to 4) cause less damage to hair (evaluated by hair texture), and exhibit excellent hair straightening effect, as compared with the case of the composition of Comparative Example 2 (i.e., formulation of Patent Document 1). Also, the samples of these Examples provide higher panelist acceptability. As shown in Table 2, when glycerin is replaced with sorbitol, damage sensation is further suppressed, and hair handling performance is improved. As is clear from these data, even when an amidoamine compound is replaced with O-[2-hydroxy-3-(trimethylammonio)propyl]hydroxyethylcellulose chloride (i.e., cationized cellulose) (Example 4), the effects of the present invention are achieved.

[Test Example 2] Combing test

<Test method>

Bleached hair samples were subjected to the treatment (2) of the actual use test of Test Example 1. Specifically, the first component (2.0 g) was applied to each of the bleached hair samples, and the hair sample was allowed to stand at 25°C for 15 minutes. Then, (i) washing with water (rinsing), and (ii) towel drying were carried out. Thereafter, the thus-dried hair sample was treated with a high-temperature styling iron set at 170°C for three seconds. Subsequently, the second component (2.0 g) was applied to the hair sample, and the hair sample was allowed to stand at 25°C for five minutes and then washed with water, followed by (iii) natural drying.

By means of a combing tester, the above-straightened hair sample was subjected to a combing test immediately after (i) washing with water (rinsing), immediately after (ii) towel drying, and immediately after (iii) natural drying. The test was carried out under the following conditions: combing and showering (10 times) (immediately after rinsing) → drying with a towel and combing (10 times) (immediately after towel drying) → drying at room temperature for two hours → combing (10 times) (immediately after natural drying). The loads measured at each timing (N = 6) was averaged.

<Results of Test Example 2>

Table 3 shows the formulations of test samples (Comparative Example 3 and Example 5) employed in the test, as well as the test results. The test results are also shown in Fig. 1. The test samples were prepared in a manner similar to that of the test samples shown in Table 2.

[Table 3]

| | Comparative Example 3 | Example 5 |
|---|---|---|
| Component | Amount (mass%) | |
| Purified water | Balance | Balance |
| Amino-modified silicone | 1.0 | 1.0 |
| Mineral oil | 1.0 | 1.0 |
| Cetyl alcohol | 5.0 | 5.0 |
| Glycerin | 10.0 | - |
| Sorbitol | - | 10.0 |
| Propylene glycol | 3.0 | 10.0 |
| Polyoxyethylene oleyl ether | 0.5 | 0.5 |
| Behentrimonium chloride | 2.5 | 1.0 |
| Stearoxyhydroxypropylamine | - | 1.0 |
| Stearic acid dimethylaminopropylamide | - | 0.5 |
| Monoethanolamine | 1.5 | 1.5 |
| Ammonium thioglycolate | 7.0 | - |
| Ammonium thiolactate | - | 7.0 |
| Diammonium dithiodiglycolate | 2.0 | 2.0 |

(continued)

| | Comparative Example 3 | Example 5 |
|---|---|---|
| Component | Amount (mass%) | |
| Pentasodium diethylenetriaminepentaacetate | Appropriate amount | Appropriate amount |
| Perfume | Appropriate amount | Appropriate amount |
| Total | 100 | 100 |
| Test results | | |
| Load immediately after rinsing (N) | 4.1 | 4.0 |
| Load immediately after towel drying (N) | 3.3 | 2.9 |
| Load immediately after natural drying (N) | 2.5 | 2.3 |

As is clear from the data of Table 2 (Fig. 1), the invention product exhibits a combing load (as measured immediately after towel drying or natural drying) lower than that of the comparative product. The objective numerical values indicate that the invention product realizes suppression of damage to hair.

[Test Example 3] Hair tensile test

<Test method>

Bleached hair samples were subjected to the treatment (2) of the actual use test of Test Example 1. Specifically, the first component (2.0 g) was applied to each of the bleached hair samples, and the hair sample was allowed to stand at 25°C for 15 minutes and then washed with water (rinsing), followed by drying with a towel. Thereafter, the thus-dried hair sample was treated with a high-temperature styling iron set at 170°C for three seconds. Subsequently, the second component (2.0 g) was applied to the hair sample, and the hair sample was allowed to stand at 25°C for five minutes and then washed with water, followed by natural drying.

The tensile strength of hair (12 hair samples) was measured (N = 6) by means of Autograph AGS-H 50N (precise universal tester, product of Shimadzu Corporation).

Specifically, the degree of hair damage was determined by use of the following formula:

the degree of hair damage = (the tensile strength of hair as measured after the treatment)/(the tensile strength of the hair as measured before the treatment).

<Results of Test Example 3>

Table 4 shows the formulations of test samples (Comparative Example 4 and Examples 6 and 7) employed in the test, as well as the test results. The test results are also shown in Fig. 2 (the vertical axis corresponds to the degree of hair damage). The test samples were prepared in a manner similar to that of the test samples shown in Table 2.

[Table 4]

| | Comparative Example 4 | Example | Example 7 |
|---|---|---|---|
| Component | Amount (mass%) | | |
| Purified water | Balance | Balance | Balance |
| Amino-modified silicone | 1.0 | 1.0 | 1.0 |
| Mineral oil | 1.5 | 1.5 | 1.5 |
| Cetyl alcohol | 6.0 | 6.0 | 6.0 |

(continued)

| | Comparative Example 4 | Example | Example 7 |
|---|---|---|---|
| Component | Amount (mass%) | | |
| Glycerin | 15.0 | 15.0 | 15.0 |
| Sorbitol | - | - | - |
| Propylene glycol | 3.0 | 3.0 | 3.0 |
| Polyoxyethylene oleyl ether | 0.5 | 0.5 | 0.5 |
| Behentrimonium chloride | 1.0 | 1.0 | 1.0 |
| Stearoxyhydroxypropylamine | 0.5 | 0.5 | 0.5 |
| Stearic acid dimethylaminooropylamide | - | 0.3 | 0.3 |
| Monoethanolamine | 1.0 | 1.0 | 1.0 |
| Ammonium thioglycolate | 3.0 | - | - |
| Ammonium thiolactate | - | 3.0 | 4.0 |
| Diammonium dithiodiglycolate | 2.0 | 2.0 | 2.0 |
| Pentasodium diethylenetriaminepentaacetate | Appropriate amount | Appropriate amount | Appropriate amount |
| Perfume | Appropriate amount | Appropriate amount | Appropriate amount |
| Total | 100 | 100 | 100 |
| Test results | | | |
| Degree of hair damage (10-minute treatment) | 0.87 | 0.91 | 0.90 |
| Degree of hair damage (20-minute treatment) | 0.82 | 0.9 | 0.90 |
| Degree of hair damage (30-minute treatment) | 0.78 | 0.84 | 0.86 |

As is clear from the data of Table 4 (Fig. 2), generally, the degree of hair damage is lower in the case of the Examples than in the case of the Comparative Example, and, even when the amount of the reducing agent is increased, virtually no increase in degree of hair damage is observed.

Next will be described formulation examples of the present composition (a two-component hair shape-controlling composition containing the present composition as a first component, or a one-component hair shape-controlling composition). In each formulation example, a composition may be prepared through a customary method.

[Example 8] Hair straightening composition (two-component type)

<First component>

| Component | Amount (mass%) |
|---|---|
| Monoammonium thioglycolate | 10 |
| Diammonium dithiodiglycolate | 3 |
| Monoethanolamine | 5 |
| Stearoxyhydroxypropylamine | 2 |
| Stearic acid diethylaminoethylamide | 1 |
| Stearyltrimonium chloride | 1 |
| Stearyl alcohol | 7 |

(continued)

| Component | Amount (mass%) |
|---|---|
| Sorbitol | 5 |
| Pentasodium diethylenetriaminepentaacetate | Appropriate amount |
| Perfume | Appropriate amount |
| Purified water | Balance |

<Second component>

| Component | Amount (mass%) |
|---|---|
| Hydrogen peroxide | 1.5 |
| Behentrimonium chloride | 2 |
| Dimethylpolysiloxane | 1 |
| Cetyl alcohol | 1 |
| Disodium hydrogenphosphate | 0.2 |
| Potassium phosphate | 0.4 |
| Sodium benzoate | 0.1 |
| Purified water | Balance |

[Example 9] Hair straightening composition (two-component type)

<First component>

| Component | Amount (mass%) |
|---|---|
| Monoammonium thiolactate | 11 |
| Diammonium dithiodiglycolate | 4 |
| Monoethanolamine | 4 |
| Stearoxyhydroxypropylamine | 1 |
| Stearic acid dimethylaminopropylamide | 1 |
| Behentrimonium chloride | 2 |
| Stearyl alcohol | 7 |
| Sorbitol | 5 |
| Propylene glycol | 5 |
| Pentasodium diethylenetriaminepentaacetate | Appropriate amount |
| Perfume | Appropriate amount |
| Purified water | Balance |

<Second component>

| Component | Amount (mass%) |
|---|---|
| Hydrogen peroxide | 1.5 |
| Behentrimonium chloride | 2 |
| Mineral oil | 0.5 |
| Cetyl alcohol | 3 |
| Disodium hydrogenphosphate | 0.2 |
| Potassium phosphate | 0.4 |

(continued)

| Component | Amount (mass%) |
|---|---|
| Sodium benzoate | 0.2 |
| Purified water | Balance |

[Example 10] Permanent waving composition (two-component type)

<First component>

| Component | Amount (mass%) |
|---|---|
| Cysteine | 8 |
| Diammonium dithiodiglycolate | 3 |
| Monoethanolamine | 5 |
| Stearoxyhydroxypropylamine | 1.5 |
| O-[2-hydroxy-3-(trimethylammonio)-propyl]hydroxyethylcellulose chloride | 1 |
| Stearyltrimonium chloride | 1 |
| Stearyl alcohol | 7 |
| Sorbitol | 5 |
| Pentasodium diethylenetriaminepentaacetate | Appropriate amount |
| Perfume | Appropriate amount |
| Purified water | Balance |

<Second component>

| Component | Amount (mass%) |
|---|---|
| Sodium bromate | 6 |
| Behentrimonium chloride | 2 |
| Dimethylpolysiloxane | 1 |
| Cetyl alcohol | 1 |
| Disodium hydrogenphosphate | 0.2 |
| Potassium phosphate | 0.4 |
| Sodium benzoate | 0.1 |
| Purified water | Balance |

[Example 11] Hair composition (one-component type)

| Component | Amount (mass%) |
|---|---|
| Monoammonium thiolactate | 7 |
| Monoethanolamine | 4 |
| Stearoxyhydroxypropylamine | 1 |
| O-[2-hydroxy-3-(trimethylammonio)-propyl]hydroxyethylcellulose chloride | 1 |
| Stearic acid dimethylaminopropylamide | 1 |
| Behentrimonium chloride | 1 |
| Stearyl alcohol | 7 |
| Cetyl alcohol | 1 |

(continued)

| Component | Amount (mass%) |
|---|---|
| Sorbitol | 10 |
| Pentasodium diethylenetriaminepentaacetate | Appropriate amount |
| Perfume | Appropriate amount |
| Purified water | Balance |

[Example 12] Hair composition (one-component type)

| Component | Amount (mass%) |
|---|---|
| Sodium pyrosulfite | 4 |
| Monoethanolamine | 3 |
| Stearoxyhydroxypropylamine | 1 |
| Stearic acid dimethylaminopropylamide | 1 |
| Behentrimonium chloride | 1 |
| Stearyl alcohol | 4 |
| Cetyl alcohol | 3 |
| Pentasodium diethylenetriaminepentaacetate | Appropriate amount |
| Perfume | Appropriate amount |
| Purified water | Balance |

## Claims

**1.** A hair shape-controlling composition comprising a reducing agent and an alkaline agent, which composition is **characterized by** comprising the following components (1) to (3):

(1) a hydroxy-ether-amine compound represented by the following formula (I) :

[F1]

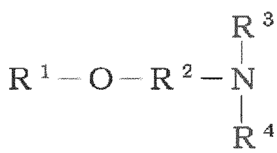

(I) ,

wherein $R^1$ represents a linear or branched C6 to C24 alkyl or alkenyl group; $R^2$ represents a linear or branched C1 to C6 hydroxyalkylene or hydroxyalkylenyl group; and each of $R^3$ and $R^4$, which may be identical to or different from each other, represents a hydrogen atom or a linear C1 to C6 alkyl group;

(2) an amidoamine compound represented by the following formula (II) and/or O-[2-hydroxy-3-(trimethylammonio)propyl]hydroxyethylcellulose chloride;

[F2]

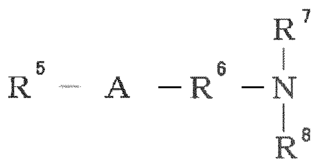

(II) ,

wherein $R^5$ represents a linear or branched C6 to C24 alkyl or alkenyl group; A represents an amide bond (-CONH-); $R^6$ represents a linear or branched C1 to C6 alkylene or alkylenyl group; and each of $R^7$ and $R^8$, which may be identical to or different from each other, represents a hydrogen atom or a linear or branched C1 to C6 alkyl group; and
(3) a higher alcohol,

**2.** The hair shape-controlling composition according to claim 1, which is a first component of a two-component hair shape-controlling composition comprising the first component containing a reducing agent and an alkaline agent, and a second component containing an oxidizing agent.

**3.** The hair shape-controlling composition according to claim 1 or 2, which further comprises a sugar alcohol.

**4.** The hair shape-controlling composition according to claim 3, wherein the sugar alcohol is sorbitol.

**5.** The hair shape-controlling composition according to any of claims 1 to 4, which further comprises a quaternary ammonium salt.

**6.** The hair shape-controlling composition according to any of claims 1 to 5, wherein the reducing agent is thiolactic acid or a salt thereof.

**7.** The hair shape-controlling composition according to any of claims 1 to 6, wherein the alkaline agent is monoethanolamine.

**8.** The hair shape-controlling composition according to any of claims 1 to 7, which is a composition for permanent wave treatment.

**9.** The hair shape-controlling composition according to any of claims 1 to 7, which is a composition for hair straightening treatment,

[Fig. 1]

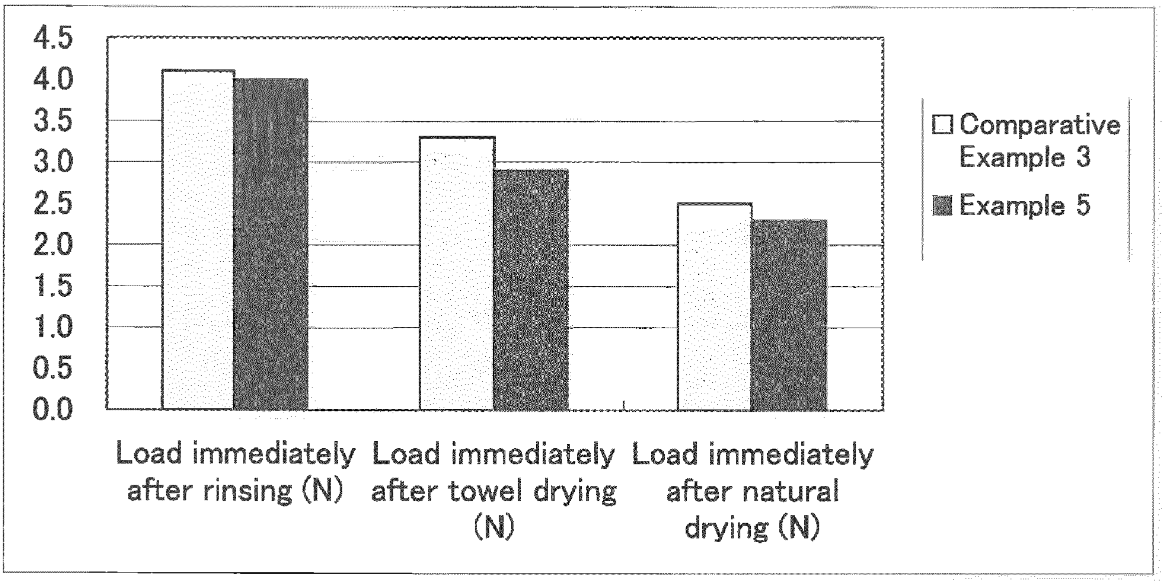
4.5
4.0
3.5
3.0
2.5
2.0
1.5
1.0
0.5
0.0
Load immediately after rinsing (N)
Load immediately after towel drying (N)
Load immediately after natural drying (N)
Comparative Example 3
Example 5

[Fig. 2]

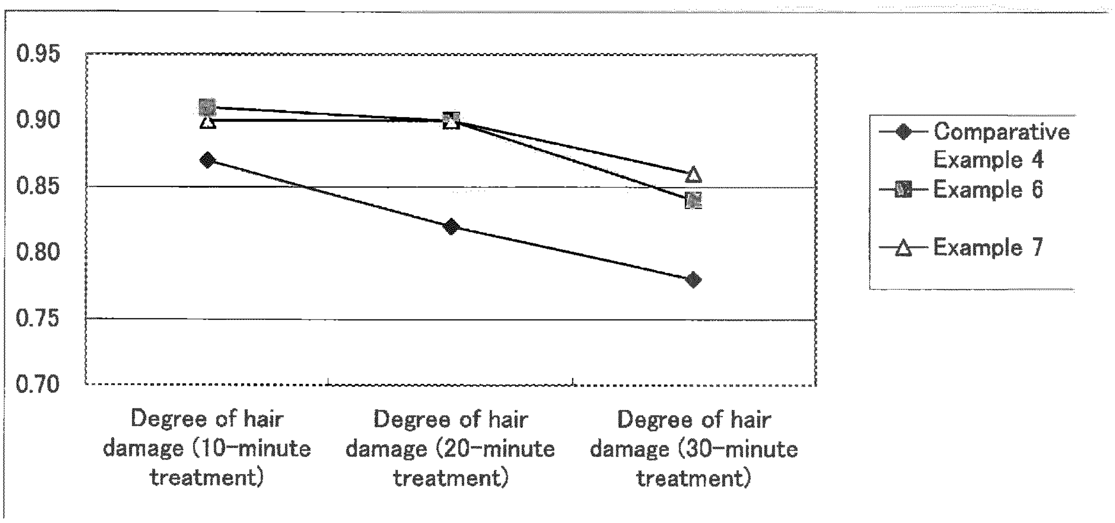
0.95
0.90
0.85
0.80
0.75
0.70
Degree of hair damage (10-minute treatment)
Degree of hair damage (20-minute treatment)
Degree of hair damage (30-minute treatment)
Comparative Example 4
Example 6
Example 7

## INTERNATIONAL SEARCH REPORT

International application No.
PCT/JP2011/053937

A. CLASSIFICATION OF SUBJECT MATTER
*A61K8/41*(2006.01)i, *A61K8/34*(2006.01)i, *A61K8/42*(2006.01)i, *A61K8/46*(2006.01)i, *A61K8/73*(2006.01)i, *A61Q5/04*(2006.01)i

According to International Patent Classification (IPC) or to both national classification and IPC

B. FIELDS SEARCHED

Minimum documentation searched (classification system followed by classification symbols)
A61K8/41, A61K8/34, A61K8/42, A61K8/46, A61K8/73, A61Q5/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
CA/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2008-231098 A (Shiseido Co., Ltd.), 02 October 2008 (02.10.2008), claims; example 21 & JP 2008-231099 A & US 2010/0111885 A1 & EP 2123250 A1 & WO 2008/102792 A1 & CN 101616655 A | 1,2,5-9<br>3,4 |
| Y | JP 2001-010936 A (Hoyu Co., Ltd.), 16 January 2001 (16.01.2001), claim 2; paragraphs [0016], [0018], [0019] (Family: none) | 3,4 |
| P,A | JP 2010-208956 A (Shiseido Co., Ltd.), 24 September 2010 (24.09.2010), claims; paragraph [0001]; table 1, examples 2, 3; table 2 & WO 2010/101253 A | 1-9 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 23 May, 2011 (23.05.11) | 31 May, 2011 (31.05.11) |

| Name and mailing address of the ISA/ | Authorized officer |
|---|---|
| Japanese Patent Office | |
| Facsimile No. | Telephone No. |

Form PCT/ISA/210 (second sheet) (July 2009)

INTERNATIONAL SEARCH REPORT

International application No.
PCT/JP2011/053937

C (Continuation). DOCUMENTS CONSIDERED TO BE RELEVANT

| Category* | Citation of document, with indication, where appropriate, of the relevant passages | Relevant to claim No. |
|---|---|---|
| A | JP 2008-189584 A (Shiseido Co., Ltd.),<br>21 August 2008 (21.08.2008),<br>paragraph [0076], prescription example 6<br>(Family: none) | 1-9 |
| A | JP 2004-323496 A (Toho Chemical Industry Co., Ltd.),<br>18 November 2004 (18.11.2004),<br>claims<br>(Family: none) | 1-9 |
| P,A | WO 2010/116940 A1 (Shiseido Co., Ltd.),<br>14 October 2010 (14.10.2010),<br>claims; paragraph [0044]<br>(Family: none) | 1-9 |
| A | JP 2004-217589 A (Shiseido Co., Ltd.),<br>05 August 2004 (05.08.2004),<br>claims<br>(Family: none) | 1-9 |
| A | JP 2006-045223 A (Kao Corp.),<br>16 February 2006 (16.02.2006),<br>claims; paragraph [0020]<br>& US 2007/0264218 A1 & EP 1782788 A1<br>& WO 2006/006541 A1 | 1-9 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2008231098 A
- JP 2004323495 A
- JP 2004323496 A